(19) Europäisches Patentamt
European Patent Office
Office européen des brevets

(11) Publication number: **0 431 200 A1**

(12) **EUROPEAN PATENT APPLICATION published in accordance with Art. 158(3) EPC**

(21) Application number: **90909848.5**

(51) Int. Cl.$^5$: **G02F 1/35**, C07C 251/24

(22) Date of filing: **28.06.90**

(86) International application number: **PCT/JP90/00846**

(87) International publication number: **WO 91/00544 (10.01.91 91/02)**

(30) Priority: **28.06.89 JP 166248/89**
**31.07.89 JP 199761/89**

(43) Date of publication of application: **12.06.91 Bulletin 91/24**

(84) Designated Contracting States: **AT BE CH DE DK ES FR GB IT LI LU NL SE**

(71) Applicant: **TORAY INDUSTRIES, INC.**
**2-1, Nihonbashi Muromachi 2-chome**
**Chuo-ku**
**Tokyo 103(JP)**

(72) Inventor: **GOTO, Tetsuya**
**40-17, Nango 2-chome Otsu**
**Shiga 520(JP)**
Inventor: **TSUNEKAWA, Tetsuya**
**103-35, Setahasimoto-cho Otsu**
**Shiga 520-21(JP)**
Inventor: **FUKUDA, Seiji**
**10-A8-32, Sonoyama 2-chome Otsu**
**Shiga 520(JP)**
Inventor: **MATAKI, Hiroshi**
**5-11, Aoyama 1-chome Otsu**
**Shiga 520(JP)**

(74) Representative: **Kador & Partner**
**Corneliusstrasse 15**
**W-8000 München 5(DE)**

(54) **QUADRATIC NONLINEAR OPTICAL ELEMENT.**

(57) A high-performance quadratic nonlinear optical element which can exhibit a large nonlinear optical effect. The element contains a part which comprises a non-centrosymmetric monoclinic crystal, belonging to the space group Cc and the point group #9, of 4'-nitrobenzylidene-3-alkanoylamino-4-methoxyaniline of formula (I) or derivatives thereof wherein at least part of the hydrogen atoms are deuterated, and which is provided with at least one substantially optically flat surface which admits or transmits rays of light having a large electric oscillation vector component in the direction of the axis of maximum absorption present in the ac face of a nuclear crystal. In formula (I) R represents a $C_1$ or $C_2$ alkyl or haloalkyl.

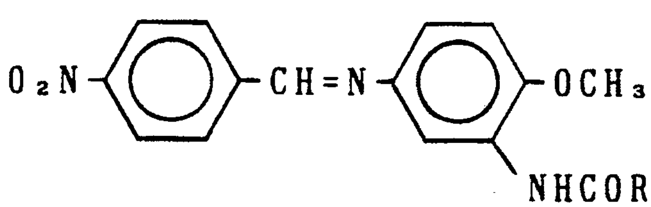

( I )

EP 0 431 200 A1

## BACKGROUND OF THE INVENTION

I. Field of the Invention

This invention relates to a second-order nonlinear optical device by which the frequency conversion effects or linear electro-optic effect (Pockels effect) is utilized. The devices are useful in the field of optical information processing and optical communications.

II. Description of the Related Art

When a strong electric field (E) such as laser beam is applied to a substance, the substance shows polarization (P) which is expressed by the following general equation:

$$P = X^{(1)}E + X^{(2)}EE + X^{(3)}EEE +$$

(wherein $X(^1)$ means linear optical susceptibility and $X^{(n)}$ (n is an integer of not less than 2) means nonlinear optical susceptibility).
The nonlinear optical effects are those expressed by the high order terms of E, i.e., terms of E of not less than square.

Examples of the second-order nonlinear optical effects include frequency conversion effects such as second harmonic generation (SHG) or parametric oscillation, and linear electro-optic effect (Pockels effect). By utilizing these effects, second-order nonlinear optical devices which are industrially important, such as frequency converters including second harmonic generators (SHG device) and parametric oscillators, or electro-optic devices such as optical switches or optical modulators can be obtained.

The second-order nonlinear optical devices include at least one optical element of an optical medium which exhibits second-order nonlinear optical effects, and which should have a substantially optically smooth surface to be impinged or transmitted by laser beam or the like. In case of electro-optic devices, the devices further comprise electrodes for applying electric field.

Employment of an optical medium which shows higher performance or higher nonlinear optical effects for constituting the second-order nonlinear optical device is advantageous because the power of the light source may be reduced, the size of the device may be made small, the voltage of the applied electric field may be reduced and the price of the device may be made less expensive.

The second-order nonlinear optical media have strong anisotropy with respect to the expression of the effects (i e., the anisotropic dependency on the optical field and external electric field ). Therefore, a device structure, namely, an element structure for allowing the optical medium to efficiently exhibit the effects is required.

Conventional nonlinear optical media include

(i) inorganic ferroelectric crystals such as lithium niobate (LN) or potassium dihydrogen phosphate (KDP) crystals;

(ii) organic crystals such as 2-methyl-4-nitroaniline (MNA); and

(iii) organic solid solution of polymers and organic molecules which can manifest second-order nonlinear optical effects (e.g., poled polymers hereinbelow described).

The nonlinear optical media (i) were firstly developed in the art and their processing technologies for an optical element or device are best known. However, their second-order nonlinear optical effects are not large. Thus, the performance of the second-order nonlinear optical devices utilizing the nonlinear optical media (i) is unsatisfactory. They are large in size and expensive. In addition, the LN crystals which show the best performance of the media (i) can be damaged by light, which is a serious problem in practical industrial applications.

The nonlinear optical media (ii) are receiving much attention recently as optical media superior to the media (i) because of the large optical nonlinearity of organic molecules due to the intramolecular $\pi$-electronic fluctuation, fast response and high resistance against laser beam.

For example, 2-methyl-4-nitroaniline (MNA) crystal was reported to have the highest nonlinear optical effects among the optical media (ii), which are larger than those exhibited by the LN crystal which is an inorganic ferroelectric nonlinear optical crystal (e.g., J. Appl. Phys., 50(4), 2523 (1979); J. Chem. Phys., 75-(3), 1509 (1981)).

However, the nonlinear optical effects of MNA crystal are not so strikingly larger than those exhibited by LN crystal. Further, MNA crystal has practical problems in that it is water-soluble and sublimated at room

temperature.

The nonlinear optical media (iii) have been developed because of the good processability of the polymers. However, their second-order nonlinear optical effects are much smaller than those typical of the optical media (ii), and at present, even smaller than those of LN crystals. This is because the density of the component (i.e., the organic molecule) exhibiting the nonlinear optical effects is lowered by the existence of the polymer and the degree of orientation of the component (pigment) exhibiting the nonlinear optical effects cannot be made so high by the poling treatment (poling treatment is detailed in Proceedings of MRS Conference, vol. 109, "Nonlinear Optical Properties of Polymers", Ed. by A. J. Heeger et al., 1988, p19). Further, the degree of orientation of the component providing the nonlinear optical effects decreases with time because of orientation relaxation, so that the performance thereof decreases with time accordingly.

Thus, all of the conventional media (i), (ii) and (iii) is unsatisfactory for realization of a second-order nonlinear optical device with high performance.

The conventional second-order nonlinear optical devices will now be described referring to the drawings.

A conventional Cherenkov SHG device 82 having a proton-exchanged waveguide 81 is shown in Fig. 8. Semiconductor laser beam impinged to the proton-exchanged waveguide is converted to its second harmonic wave with the guided-wave propagation and simultaneously radiated to the outside of the waveguide. It was reported that the maximum output power of the second harmonic wave 85 (420 nm) was about 1 mW even when a special high power semiconductor laser 86 (fundamental wavelength 840 nm) with a power of as high as 80 mW was used as a light source. A higher output power cannot be expected with the use of LN crystal of not large frequency conversion effect, although an output power of several mW is required for practical applications. Further, the size of the device is large as long as 6 mm.

To provide an SHG device which can output a power of several mW or more using a common semiconductor laser with an output power of about 40 mW, a nonlinear optical medium with much higher performance is required.

A conventional Y-branch interferometer using an LN crystal as the nonlinear optical medium, which was reported by Auracher et al., in Wave Electron. 4, 129 (1980) is shown in Fig. 9.

An input light 91 guided from one side to the other is equally divided into two waves at a Y-junction. The phases of a guided wave 92 and a guided wave 93 were opposingly shifted by the refractive index change induced by the electric field applied between the parallel electrodes. When the guided waves 92 and 93 are joined at the other Y-junction, the interference occurs because of their shifted phases, and an output light 94 is modulated. Thus, the light modulation is carried out by the electric field applied between the electrodes.

With this conventional device, it was reported that the half-wave voltage was about 4V and the maximum bandwidth was 1.3 GHz when the wavelength of the guided-wave was 633 nm. The length of the electrode is as long as 6 mm, so that the demand to promote degree of integration in the device cannot be satisfied by using LN crystal of small linear electro-optic effect.

Fig. 10 is a schematic view of an intensity modulation electro-optic device (light intensity modulator), for explaining the principle of the operation of an electro-optic device utilizing linear electro-optic effect (Pockels effect).

An electro-optic device 101 composed of a birefringent crystal, and electrodes 104 and 105 are placed between a polarizer 102 and an analyzer 103. Here, the electro-optic element 101 is disposed such that its optic main axes (y and z) of the crystal form an angle of 45° to the crossed polarizers. When a linearly polarized light is impinged to the electro-optic element 101, the impinged light is decomposed into an ordinary light component (y) and an extraordinary light component (z), and each light component is independently transmitted through the crystal. The phases of the light components are shifted by the static birefringence intrinsic to the crystal and the electric field-induced birefringence generated when an electric field is applied between the electrodes. Only the component of the resulting ellipsoidal polarized light along the polarization axis of the analyzer can transmit the analyzer, so that the light intensity is modulated depending on the intensity of the applied electric field.

The relationship between the intensities of the input light ($I_0$) and the output light (I) is expressed by the equation

$$I = I_0 \sin^2(\phi/2) \quad (1)$$

wherein $\phi$ means the phase shift between the ordinary light and the extraordinary light, which is expressed by the equation

$\phi = (2\pi \ell/\lambda) \times (\delta - 1\ 2 \times n^3 r_{eff} E)$ (2)

wherein $\ell$ means the light path length, $\lambda$ means the wavelength of the light, $\delta$ means the static birefringence, n means the refractive index, $r_{eff}$ means the effective electro-optic coefficient, and E means the intensity of the electric field.

In the above equation (2), $1/2 \times n^3 r_{eff} E$ represents the electric field-induced birefringence. Thus, it can be seen from the above equations that the intensity of the output light (I) can be controlled within the range of

$0 \leqq I \leqq I_0$.

The intensity of the electric field E which is required for shifting the phase difference $\phi$ by $\pi$, i.e., the voltage $V_\pi$ is an important parameter called half-wave voltage, which is expressed by the equation of

$V_\pi = \lambda \cdot d/(n^3 r_{eff} \cdot \ell)$ (3)

wherein d represents the distance between electrodes.

Thus, $n^3 r_{eff}/2$ is an important factor which determines the performance of the device, which is called a figure of merit for the half-wave voltage. Use of the optical medium with the larger figure of merit makes the driving voltage lower or the size of the device smaller.

The figure of merit ($n^3 r_{33}/2$) of the LN crystal which has been the major object for constructing electro-optic devices is about 120 pm/V, which is not so large. The reason why a truly practical light modulator or optical switch has not yet been realized in spite of much expectation is concentrated to that a nonlinear optical medium with large figure of merit has not yet been found.

Nonlinear optical media and electro-optic devices other than explained above are detailed in, for example, Springer Proceedings in Physics 18, "Electro-optic and Photorefractive Materials" Ed. by P. Günter, 1987, pp2 - 17, pp150 - 158, pp159 - 164, and in Hiroshi NISHIHARA et al., "Integrated Optical Circuit", published by Ohm Co., Ltd., February 25, 1985.

Thus, studies aiming at providing a nonlinear optical medium of high performance to realize practically useful second-order nonlinear optical device are actively made with centering on the organic crystals.

## SUMMARY OF THE INVENTION

Accordingly, an object of the present invention is to provide high performance second-order nonlinear optical devices such as frequency converters or electro-optic devices by discovering a high performance nonlinear optical medium which exhibits large second-order nonlinear optical effects and has acceptable stability and processability, and by properly shaping the medium to form an optical element so that the nonlinear effects of the medium can be effectively utilized.

The present inventors intensively studied to discover a nonlinear optical medium with which high performance second-order nonlinear optical devices can be constructed, by measuring the SHG intensities by Maker fringe method and by measuring the linear electro-optic effect. As a result, the present inventors found that a specific crystal of 4'-nitrobenzylidene-3-alkanoylamino-4-methoxyaniline or a derivative thereof in which at least one hydrogen atom is substituted with deuterium atom has extremely large second-order nonlinear susceptibility $X^{(2)}$.

That is, the present invention provides a second-order nonlinear optical device comprising a non-centrosymmetric monoclinic crystal of 4'-nitrobenzylidene-3-alkanoylamino-4-methoxyaniline of the formula [I]

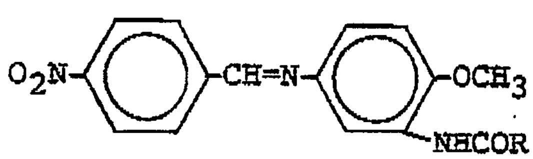

[I]

(wherein R represents a $C_1$ or $C_2$ alkyl or haloalkyl) or a derivative thereof in which at least one hydrogen atom is substituted with deuterium atom, said monoclinic crystal belonging to space group Cc, point group #9, said optical element having at least one substantially optically smooth surface for impinging, transmitting

and/or propagating a light having a main component of the optical wave with polarization along the axis of absorbing in the longest wavelengths, said axis being in the ac plane of said monoclinic crystal.

By the present invention, high performance second-order nonlinear optical devices such as frequency converters including SHG device or light modulators including optical switch were provided by utilizing 4'-nitrobenzylidene-3-alkanoylamino-4-methoxyaniline crystal which exhibits larger second-order nonlinear optical effects such as frequency conversion effect or linear electro-optic effect than the conventional second-order nonlinear optical media, and also, has an acceptable stability and processability. Thus, the present invention will largely contribute to the development of optical information processing and optical communications.

## BRIEF DESCRIPTION OF THE DRAWINGS

Fig. 1a schematically shows the structure of MNBA crystal which is useful as a second-order nonlinear optical medium;

Fig. 1b shows the structure cf MNBA-Et crystal which is useful as a second-order nonlinear optical medium;

Fig. 2 shows the morphology of a thin film MNBA-Et single crystal which has a smooth (0 1 0) face;

Fig. 3a schematically shows a constitution of an optical modulator utilizing an electro-optic element made of MNBA or MNBA-Et thin film single crystal;

Fig. 3b shows the relationship between the applied voltage and the detected output voltage of a photodetector corresponding to the output light intensity, which was depicted by an oscilloscope;

Fig. 4 schematically shows the constitution of an SHG device utilizing a plate-shaped MNBA-Et single crystal;

Fig. 5a schematically shows the constitution of a branched interferometer type optical modulator having a MNBA crystal waveguide;

Fig. 5b is a schematic cross sectional view of a branched interferometer type optical modulator having MNBA crystal waveguide;

Fig. 6 shows the relationship between the voltage applied to the branched interferometer type optical modulator and the detected output voltage of a photodetector corresponding to the output light intensity, which was depicted by an oscilloscope;

Fig. 7 schematically shows the constitution of a total internal reflection (TIR) type optical switch having MNBA crystal waveguide;

Figs. 8a and 8b schematically show the constitution of a conventional Cherenkov SHG device utilizing LN crystal;

Fig. 9 schematically shows the constitution of a conventional branched interferometer type optical modulator utilizing LN crystal; and

Fig. 10 is a view for explaining the principle of the operation of an electro-optic device utilizing linear electro-optic effect.

## DETAILED DESCRIPTION OF THE PREFERRED EMBODIMENTS

Preferred examples of non-centrosymmetric monoclinic single crystals of the 4'-nitrobenzylidene-3-alkanoylamino-4-methoxyaniline represented by the above-described formula [I], which may be used in the present invention include 4'-nitrobenzylidene-3-acetoamino-4-methoxyaniline (MNBA), 4'-nitrobenzylidene-3-ethylcarbonylamino-4-methoxyaniline (MNBA-Et), 4'-nitrobenzylidene-3-chloroacetoamino-4-methoxyaniline (MNBA-Cl), 4'-nitrobenzylidene-3-bromoacetoamino-4-methoxyaniline (MNBA-Br) and 4'-nitrobenzylidene-3-($\beta$-chloroethyl)carbonylamino-4-methoxyaniline (MNBA-ClEt), as well as their derivatives in which at least one hydrogen atom is substituted with dueterium atom.

It was found that all of the crystals of the 4'-nitrobenzylidene-3-alkanoylamino-4-methoxyaniline of the formula [I] are non-centrosymmetric monoclinic crystals belonging to space group Cc, point group #9. There is no other examples that all of the derivatives of a compound form non-centrosymmetric monoclinic crystals belonging to the same space group and point group. It most frequently occurs that if the molecular structures are little different, the crystals made of the molecules belong to utterly different space groups and point groups, and the non-centrosymmetry of a crystal of a derivative, which is indispensable to the optical nonlinearity, is often lost in another derivative.

It should be noted, however, that each monoclinic single crystal of 4'-nitrobenzylidene-3-alkanoylamino-4-methoxyaniline has specific lattice constants. For example, MNBA forms a non-centrosymmetric monoclinic crystal shown in Fig. 1a belonging to space group Cc, point group #9, and has the following lattice constants at 23±1 °C:

$$a = 8.3814(9)\text{Å} \quad \beta = 115.336(5)^\circ$$
$$b = 26.838(4)\text{Å} \quad z = 4$$
$$c = 7.4871(5)\text{Å}$$

MNBA-Et forms a non-centrosymmetric monoclinic crystal shown in Fig. 1b belonging to space group Cc, point group #9, and has the following lattice constants at 23±1 °C:

$$a = 8.276(2)\text{Å} \quad \beta = 115.27(1)^\circ$$
$$b = 28.057(3)\text{Å} \quad z = 4$$
$$c = 7.651(1)\text{Å}$$

(It should be noted that the above-described lattice constants may fluctuate depending on the measurement conditions within the range of several percent, as is recognized by the example differences between the works reported by B.F. Levine et al., J. Appl. Phys. 50(4), 2523 (1979); and G.F. Lipscomb et al., (J. Chem. Phys. 75(3), 1509 (1981)).

The non-centrosymmetric monoclinic single crystal of 4'-nitrobenzylidene-3-alkanoylamino-methoxyaniline may be obtained easily by the solution growth method using acetone or DMF or others as the solvent, or by melt growth method, which are well-known in the art.

The method of providing a high performance second-order nonlinear optical device such as frequency converter and electro-optic devices by properly shaping the above-described single crystal of 4'-nitrobenzylidene-3-alkanoylamino-4-methoxyaniline to form an optical element, by which the nonlinear optical effect thereof can be effectively utilized, will now be described.

Taking the crystallographic b axis of the 4'-nitrobenzylidene-3-alkanoylamino-4-methoxyaniline crystal as y axis, the axis of absorbing in the longest wavelengths in the ac plane as x axis, and the axis perpendicular to these axes as z axis, the second-order nonlinear optical susceptibility matrix is expressed by the following formula (4) based on the symmetry (point group, m):

$$\begin{pmatrix} \chi^{(2)}_{11} & \chi^{(2)}_{12} & \chi^{(2)}_{13} & 0 & \chi^{(2)}_{15} & 0 \\ 0 & 0 & 0 & \chi^{(2)}_{24} & 0 & \chi^{(2)}_{26} \\ \chi^{(2)}_{31} & \chi^{(2)}_{32} & \chi^{(2)}_{33} & 0 & \chi^{(2)}_{35} & 0 \end{pmatrix} \quad \cdots (4)$$

Judging from the crystal structures,

$$\chi^{(2)}_{11}$$

should be the maximum susceptibility.

Therefore, it was deduced by the present inventors that (i) impinging, transmitting and/or propagating a light having a main component of the optical wave with polarization in the ac plane of the crystal is preferred because large nonlinear optical effects may be obtained; and (ii) impinging or transmitting, and/or propagating a light having a main component of the optical wave with pclarization along the x axis is most preferred. Further, when the impinging or transmitting direction of a light satisfies above-described (i) and (ii), it was further deduced by the present inventors that (iii) applying an electric field having a main component in the ac plane of the crystal is preferred because a large linear electro-optic effect may be

obtained; and (iv) applying an electric field having a main component in the x axis is most preferred.

The present inventors discovered the relationships among the second-order nonlinear susceptibility, anisotropy and crystal structure by the measurement of SHG intensities by Maker fringe method (detailed in Electron. Lett. vol. 23, No. 11, 595 (1987)) and the measurement of dependency on the direction of the electric field in optical modulators. As a result, the present inventors proved the above-mentioned deduction and substantiated the direction of the substantially optically smooth surface which the second-order nonlinear optical element should have, and completed the present invention.

In the measurement of SHG intensities, it was found that MNBA crystal has a second-order nonlinear susceptibility

$$\chi^{(2)}_{11}$$

of about 5.0 x $10^{-6}$ esu which is about four times larger than that of the MNA crystal which has the largest

$$\chi^{(2)}_{11}$$

among the conventional crystals, and that MNBA-Et crystal has a second-order nonlinear susceptibility

$$\chi^{(2)}_{11}$$

of about 3.9 x $10^{-6}$ esu which is about three times larger than that of the MNA crystal. This means that the SHG device utilizing the MNBA crystal or MNBA-Et crystal has a frequency converting ability per a unit length Of about 18 times or about 11 times larger than that of the SHG device utilizing the MNA crystal when the impinging light density is the same. Further, when compared with the SHG device utilizing the LN crystal, the frequency converting ability thereof is as large as about 900 times or about 560 times, respectively.

Therefore, in a case where a SHG device with a certain frequency conversion efficiently is constructed, the power of the light source can be reduced and the device can be made small if MNBA or MNBA-Et crystal is utilized.

Further, the figure of merit of the linear electro-optic effect $|n_1{}^3r_{11} - n_3{}^3r_{31}|/2$ was determined by constructing optical modulators. As a result, the figure of merit of the optical modulator utilizing the MNBA crystal was about 930 pm/V and that of the optical modulator utilizing the MNBA-Et crystal was about 700 pm/V, which are about 3 times and about 2.3 times, respectively, as large as that of the optical modulator utilizing the MNA crystal which gives the largest figure of merit. When compared with the figure of merit given by an optical modulator utilizing the LN crystal, these values are as large as about 8 times and about 6 times, respectively.

Therefore, by utilizing the MNBA or MNBA-Et crystal, a high performance electro-optic device whose size is small and which may be driven with a low voltage can be attained.

It was also proved that other 4'-nitrobenzylidene-3-alkanoylamino-4-methoxyaniline crystals can provide excellent frequency conversion devices and electro-optic devices as well as the MNBA and MNBA-Et crystals.

Thus, by providing the 4'-nitrobenzylidene-3-alkanoylamino-4-methoxyaniline crystals having the above-described specific crystal structure with a substantially optically smooth surface for impinging, transmitting, and/or propagating a light having a main component of the optical wave with polarization in the direction mentioned in (i)(x axis) so as to construct a second-order nonlinear optical element, an extremely useful second-order nonlinear optical device which exhibits large second-order nonlinear optical effects which cannot be attained by the conventional devices, is provided.

To impinge, transmit, and/or propagate a light having a main component of the optical wave with polarization herein means that a light is impinged, transmitted, and/or propagated in such manner that the main component of the light, when the light is divided into two eigenmode of the optical wave with polarization along the optical main axes of the crystal, is impinged, transmitted, and/or propagated substantially along the direction stated in (i). In the case where the light is a linearly polarized light (a light which has only one component of the optical wave), the light is impinged, transmitted, and/or propagated in such manner that this component of the optical wave is coincident with the direction mentioned in (i).

As the substantially optically smooth surface for impinging, transmitting, and/or propagating light, the as-grown surface obtained in the solution growth method may be used in some cases. Alternatively, the surface may be obtained by growing the crystal between a pair of smooth glass plates or in a smooth template in a solution growth method. Further, the as-grown faces obtained in the melt growth method may also be employed as the smooth surface. Still further, the smooth surface may be formed by the post-processing of the crystal, such as by cutting, cleaving or polishing.

The substantially optically smooth surface is a low scattering surface and may be a flat surface, curved surface or in some cases, a grating surface having a regular pattern of unevenness. That is, any surface can be used as an optically smooth surface if the impingement, transmission, propagation, reflection or emission of a light can be accomplished with a low loss.

The (0 1 0) face (the largest face) which is a naturally grown face is preferred as the substantially optically smooth surface satisfying the requirements mentioned in (i).

Further, as shown in Fig. 2, if a crystal is grown between a pair of plates by the solution growth method, a thin film single crystal having (0 1 0) face as the substantially optically smooth surface may be obtained. This crystal is especially useful for constructing the elements in which a light is guided in the TE mode.

The second-order nonlinear optical element having a pair of electrodes for applying an electric field having a main component in the direction mentioned in (iv) (x axis) is very useful as an electro-optic device.

To apply an electric field having a main component herein means that an electric field is applied in such manner that the main component of the electric field is substantially coincident with the direction mentioned in (iv) in a portion of the nonlinear optical crystal which is interacting with the light.

To construct a second-order nonlinear optical device utilizing the 4'-nitrobenzylidene-3-alkanoylamino-4-methoxyaniline crystal, it is necessary to know the x axis mentioned in (i) and (iv). Although this may be carried out by the conventional X-ray diffraction analysis, the present inventors developed a new method for simple determination of the axis.

That is, since the direction of Σ axis of the 4'-nitrobenzylidene-3-alkanoylamino-4-methoxyaniline crystal is the direction of the axis of absorbing in the longest wavelengths, the x axis can be determined by observing the crystal under a crossed Nicol (i.e., the polarizer and the analyzer are perpendicular to each other) with a polarization microscope so as to find the refractive thus optic main axes x, y and z, and then observing the crystal under a paralleled Nicol (i.e., the polarizer and the analyzer are parallel) so as to find the axis having an absorption in the longest wavelength region (i.e., most colored axis). The axis thus found is the x axis.

4'-nitrobenzylidene-3-alkanoylamino-4-methoxyaniline crystals have (0 1 0) face as a naturally grown face and the x axis exists in this face.

Therefore, the electrodes may be provided on the (0 1 0) face. Examples 2 and 4 hereinbelow described are the examples of this mode.

The device wherein a pair of electrodes are provided on (0 1 0) face or (0 -1 0) face of a thin film single crystal obtained by growing the crystal between a pair of plates by the solution growth method is one of the preferred modes. In this case, it is preferred to further process the crystal element to have a three dimensional waveguide (channel waveguide) for waveguide type electro-optic device since the longest nonlinear optical effect is manifested in the TE waveguiding mode. The examples of this type of devices are described in Examples 6 and 7 hereinbelow described.

With the SHG device described in Example 5 below, when a Q switched Nd:YAG laser (1064 nm) is guided in TE mode, that is, when the laser is impinged in the passive waveguide made of tantalum pentoxide ($Ta_2O_5$) formed on (0 1 0) face in such manner that the main component of the optical wave propagates in the plane perpendicular to the b axis of the crystal, SHG light (532 nm) was emitted from the crystal side.

In this example, the substantially optically smooth surface is (0 1 0) face contacting the passive waveguide, and this device is operated by the evanescent wave having a main component of the optical wave in the plane perpendicular to the b axis of the crystal, which evanescent wave leaks from the waveguide into the crystal. Thus, this is an example wherein the device has only one substantially optically smooth surface.

As described above, by giving an element structure by which the large nonlinear optical effects can be expressed to the 4'-nitrobenzylidene-3-alkanoylamino-4-methoxyaniline crystal having a specific structure, a high performance second-order nonlinear optical device which hitherto cannot be attained can be provided, which is extremely useful in the industry.

It should be noted that the method for growing the 4'-nitrobenzylidene-3-alkanoylamino-4-methoxyaniline crystal with a specific structure is not limited to the methods described above.

The second-order nonlinear optical device of the present invention is not limited to the above-described

devices such as frequency converters or optical switches, but a wide variety of devices can be constructed in accordance with the present invention.

Further, the element of the device in the present invention may be constituted by the crystal alone or may comprise other constituents such as electrodes, protective layers, waveguide layers, anti-reflecting coating layers, or the like. The element of the device in the present invention may also be formed on a substrate other than a glass substrate. Further, the element of the device in the present invention may be a part of an apparatus. Anyway, as far as a device comprises a crystal element of 4'-nitrobenzylidene-3-alkanoylamino-4-methoxyaniline having the above-described specific structure, which crystal has at least one substantially optically smooth surface for impinging, transmitting, and/or propagating a light having a main component of the optical wave with polarization along the axis of absorbing in the longest wavelengths, the device can function as a nonlinear optical device and the device is within the scope of the present invention.

The present invention will now be described in more detail by way of examples thereof. Although the examples of the devices comprising MNBA or MNBA-Et crystal elements are described hereinbelow, it should be understood that other crystals defined in the present invention may be equally employed and the following examples should not be interpreted in any restrictive way.

Example 1

Synthesis, Purification, Physical Properties and Evaluation of Stability of 4'-nitrobenzylidene-3-acetoamino-4-methoxyaniline (MNBA) and 4'-nitrobenzylidene-3-ethylcarbonylamino-4-methoxyaniline (MNBA-Et)

I) MNBA

1-1) Synthesis and Purification of MNBA

[Synthesis 1]

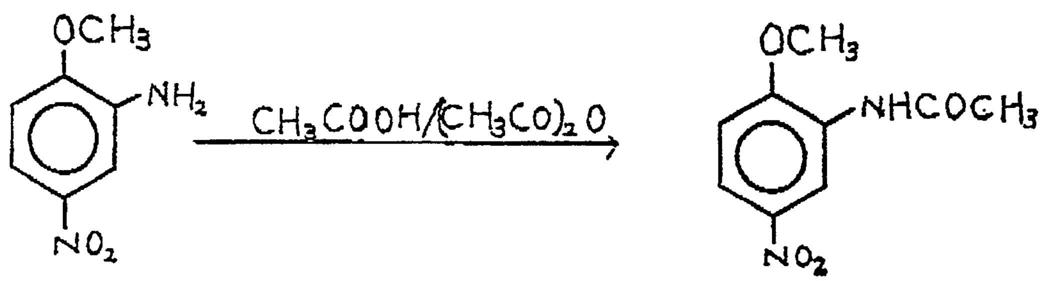


About 30 ml of acetic acid/acetic anhydride (1:1) and 8.40 g (50 mmol) of 2-amino-4-nitroanisole were placed in a 200 ml three-necked flask equipped with a reflux condenser, a nitrogen inlet tube and a dropping funnel, and the resulting mixture was stirred for about 5 hours at room temperature. The mixture was poured into a cold water. Pale yellow crude crystals were obtained and were dried in vacuum under heat to obtain 9.83 g of the desired product (yield: 93.5%).

[Synthesis 2]

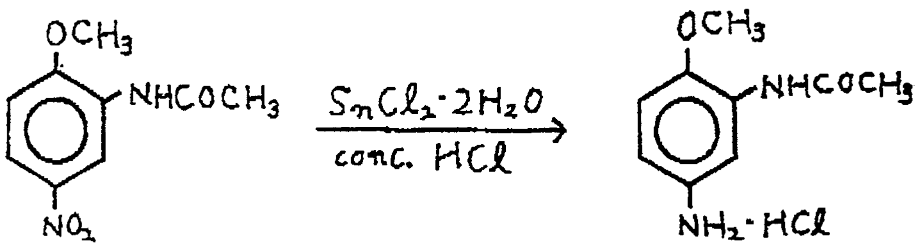

In about 30 ml of concentrated hydrochloric acid, 20.2 g (90 mmol) of stannous chloride dihydrate was dissolved. To the resulting mixture, 6.31 g (30 mmol) of 4-nitro-2-acetylaminoanisole obtained in Synthesis 1 was added under vigorous agitation and the resultant was stirred at room temperature. The color of the yellow reaction mixture turned to pale pink with evolving reaction heat.

After about two hours, the pale pink reaction mixture was filtered and washed with cold water to obtain white crude crystals. The obtained crude crystals were dried in vacuum to obtain 2.71 g of the desired product (yield: 41.7%)

[Synthesis 3]

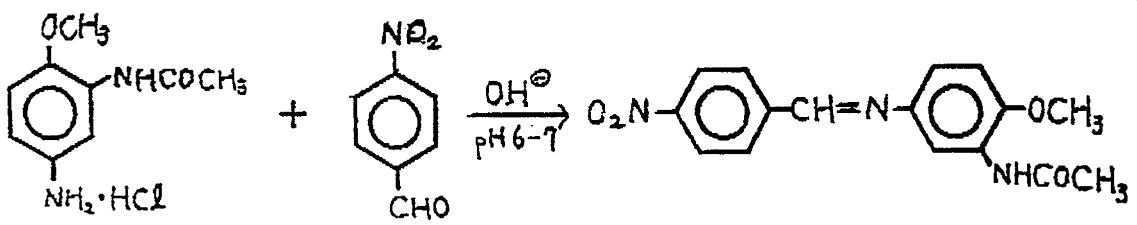


In a 200 ml three-necked flask equipped with a reflux condenser and a magnetic stirrer, 2.16 g (10 mmol) of 3-acetylamino-4-methoxyaniline hydrochloride obtained in Synthesis 2 and 1.56 g (10 mmol) of p-nitrobenzaldehyde were placed. About 60 ml of ethanol/water (2:1) as a reaction solvent was added to the mixture and the resulting mixture was stirred for 10 minutes at room temperature.

An aqueous sodium hydroxide solution was then gradually added to the mixture to a pH of 6 - 7, and the reaction mixture turned yellow brown. The mixture was stirred for another about three hours at room temperature.

After confirming the completion of the reaction by thin layer chromatography using chloroform as the developing solvent, the stirring was stopped. The precipitated crude product was collected by filtration and was washed with cold ethanol.

The yellow brown crude product thus obtained was dissolved in acetone and the insoluble materials were removed, followed by removal of the solvent by using a rotary evaporator to obtain yellow orange crude crystals.

The crude crystals thus obtained were recrystallized from chloroform/benzene (1/2) to obtain yellow crystals. The crystals thus obtained were collected by filtration and were dried in vacuum to obtain 1.75 g of the desired product (yield 56.0%).

1-2) Physical Properties of MNBA Crystals and Evaluation of Stability

Crystals of MNBA were grown by the solution method using various solvents, melt method or gas phase method (sublimation method). The crystals were pulverized and the particles with a particle size of about 100 μm were used as sample for SHG powder test.

The light source used in the measurement was 1064 nm of Nd:YAG laser, and the irradiation conditions were pulse width of 200 ns, repetition of 10 Hz and peak power density of about 30 $MW/cm^2$. The relative SHG intensity based on the SHG intensity of urea is shown in Table 1a.

As is apparent from the results, MNBA does not have the so called polymorphism. That is, crystals of the same structure can be stably prepared irrespective of the method for growing crystals.

The physical properties and stability of MNBA were examined.

The melting point of MNBA crystal was about 193° C.

Relatively largely grown yellow plate crystals (4 x 5 x 2 $mm^3$) were placed in glass test tubes at room temperature. One of the test tubes was sealed tightly and another test tube was left open, and the test tubes were left to stand. After 30 days, the weight of the either test tubes did not change and the glass wall of the sealed test tube was not colored yellow. Thus, it was proved that this crystal does not sublimate at room temperature. After the leaving to stand, the crystals were pulverized and the SHG intensities were examined by the powder method. The SHG intensities did not change before and after the leaving to stand. By this, it was proved that the optical nonlinearity of this crystal at room temperature is stable.

The above-described test of sublimation and of stability of the optical nonlinearity were checked at 100° C. The weight and the SHG intensity obtained by the powder method did not change before and after

the leaving to stand, so that it was proved that the crystal is stable also at 100 °C.

The weighed crystals were placed in a vacuum ampoule and the ampoule was connected to a vacuum line. The air in the ampoule was pumped out and the sublimation of the crystals at room temperature at $10^{-5}$ Torr was checked. After 24 hours, the weight of the crystals did not change and it was found that these crystals do not sublimate at room temperature even under a high vacuum. Under the high vacuum at elevating temperature, the sublimation was first observed at 150 °C.

The crystals and water were placed in a glass test tube and the tube was tightly sealed, followed by leaving to stand at room temperature. Even after 30 days, the water was not colored. Thus, the crystals have no solubility in water and are stable.

1-3) Analysis of Structure of MNBA Crystal by X-ray Diffraction Analysis and Optical Properties Thereof

The structure of the MNBA which is useful as a second-order nonlinear optical medium was analyzed to identify the structure.

As the sample single crystals, the prismatic yellow crystals (0.550 x 0.500 x 0.500 $mm^3$) obtained by slow evaporation from an acetone solution were used. The measurement was carried out using Rigaku AFC6R diffractometer employing CuKα ray (λ = 1.54178Å).

The results are shown in Table 2a and in Fig. 1a.

In the crystal optics, the monoclinic crystals belong to optical biaxial crystals, and all the three principal refractive indices have different value each other. By observing an interference image by a conoscope, it was proved that the direction of one of the axes of principal refractive indices (optic main axis) coincides with the crystallographic b axis. Therefore, other two optic main axes are in the ac plane.

Thus, it was identified that MNBA crystal useful as a second-order nonlinear optical medium has the above-described structure and optical properties.

II) MNBA-ET

2-1) Synthesis and Purification of MNBA-Et

[Synthesis 1]

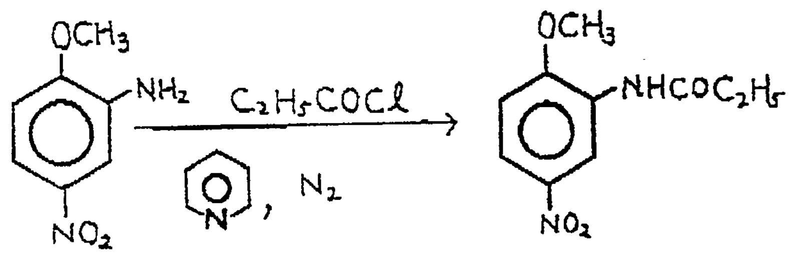


In a 300 ml three-necked flask equipped with a reflux condenser, a nitrogen inlet tube and a dropping funnel, about 100 ml of 1,2-dichloroethane, 8.40 g (50 mmol) of 2-amino-4-nitroanisole and 1.2 ml of pyridine were placed. The mixture was stirred with a magnetic stirrer under nitrogen atmosphere for about 10 minutes at room temperature.

The resulting mixture was cooled in iced water bath and about 100 ml of 1,2-dichloroethane containing 4.63 g (50 mmol) of propionyl chloride was added thereto during about 15 minutes using the dropping funnel. The orange reaction mixture turned light yellow immediately.

The temperature of the reaction mixture was raised to room temperature and then to about 50 °C, and was stirred for about 5 hours at this temperature.

After confirming the completion of the reaction by thin layer chromatography, the reaction mixture was transferred to a separation funnel and about 150 ml of chloroform was added to completely dissolve the precipitated crystals. The mixture was then well washed with about 1N diluted hydrochloric acid and the resultant was dried over anhydrous sodium sulfate. The solvent was then removed by using a rotary evaporator to obtain light yellow crude crystals. The crude crystals were then dried by heating in vacuum to obtain 10.26 g of the desired product (yield: 91.5%).

[Synthesis 2]

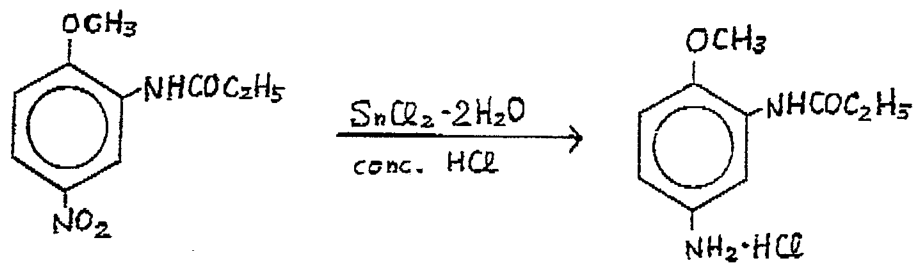


In about 30 ml of concentrated hydrochloric acid, 20.2 g (90 mmol) of stannous chloride dihydrate was dissolved. To the resulting mixture, 6.73 g (30 mmol) of 4-nitro-2-ethylcarbonylaminoanisole obtained in Synthesis 1 was added under vigorous agitation and the resultant was stirred at room temperature. The color of the yellow reaction mixture turned pale pink with evolving reaction heat.

After about two hours, the pale pink reaction mixture was filtered and washed with cold water to obtain white crude crystals. The obtained crude crystals were dried in vacuum to obtain 2.48 g of the desired product (yield: 35.8%)

[Synthesis 3]

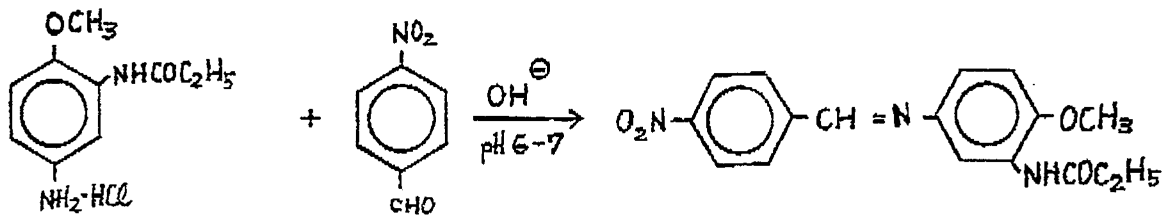


In a 200 ml three-necked flask equipped with a reflux condenser and a magnetic stirrer, 2.31 g (10 mmol) of 3-ethylcarbonylamino-4-methoxyaniline hydrochloride obtained in Synthesis 2 and 1.56 g (10 mmol) of p-nitrobenzaldehydewere placed. About 60 ml of ethanol/water (2:1) as a reaction solvent was added to the mixture and the resulting mixture was stirred for 10 minutes at room temperature.

An aqueous sodium hydroxide solution was then gradually added to the mixture to a pH of 6 - 7, and the reaction mixture turned yellow brown. The mixture was stirred for another about three hours at room temperature.

After confirming the completion of the reaction by thin layer chromatography using chloroform as the developing solvent, the stirring was stopped. The precipitated crude product was collected by filtration and was washed with cold ethanol.

The yellow brown crude product thus obtained was dissolved in acetone and the insoluble materials were removed, followed by removal of the solvent by using a rotary evaporator to obtain yellow orange crude crystals.

The crude crystals thus obtained were recrystallized from chloroform/benzene (1/3) to obtain yellow crystals. The crystals thus obtained were collected by filtration and were dried in vacuum to obtain 2.08 g of the desired product (yield 63.5%).

2-2) Physical Properties of MNBA-ET Crystals and Evaluation of Stability

Crystals of MNBA-Et were grown by the solution method using various solvents, melt method or gas phase method (sublimation method). The crystals were pulverized and the particles with a particle size of about 100 $\mu$m were used as sample for SHG powder test.

The measurement was conducted in the same manner as in the measurement for MNBA crystals. The relative SHG intensity based on the SHG intensity of urea is shown in Table 1b.

As is apparent from the results, MNBA-Et does not have the so called polymorphism.

The physical properties and stability of MNBA-Et were examined.

The melting point of MNBA-Et crystal was about 169 °C.

Relatively largely grown yellow plate crystals (5 x 4 x 3 $mm^3$) were placed in glass test tubes at room temperature. One of the test tubes was sealed tightly and another test tube was left open, and the test tubes were left to stand. After 30 days, the weight of the either test tubes did not change and the glass wall of the sealed test tube was not colored yellow. Thus, it was proved that this crystal does not sublimate at room temperature. After the leaving to stand, the crystals were pulverized and the SHG intensities were examined by the powder method. The SHG intensities did not change before and after the leaving to stand. By this, it was proved that the optical nonlinearity of this crystal at room temperature is stable.

The above-described test of sublimation and of stability of the optical nonlinearity were checked at 80 °C. The weight and the SHG intensity obtained by the powder method did not change before and after the leaving to stand, so that it was proved that the crystal was stable also at 80 °C.

The weighed crystals were placed in a vacuum ampoule and the ampoule was connected to a vacuum line. The air in the tube was pumped out and the sublimation of the crystals at room temperature and at $10^{-5}$ Torr was checked. After 24 hours, the weight of the crystals did not change and it was found that these crystals do not sublimate at room temperature even under a high vacuum.

The crystals and water were placed in a glass test tube and the tube was tightly sealed, followed by leaving to stand at room temperature. Even after 30 day, the water was not colored. Thus, the crystals have no solubility in water and are stable.

2-3) Analysis of Structure of MNBA-Et Crystal by X-ray Diffraction Analysis and Optical Properties Thereof

The structure of the MNBA-Et which is useful as a second-order nonlinear optical medium was analyzed to identify the structure.

As the sample single crystals, the prismatic yellow crystals (0.450 x 0.450 x 0.370 $mm^3$) obtained by slow evaporation from an acetone solution were used. The measurement was carried out using Rigaku AFC6R diffractometer employing MoKα ray (λ = 1.71069Å).

The results are shown in Table 2b and in Fig. 1b.

By observing an interference image by a conoscope, it was proved that the direction of one of the axes of principal refractive indices (optic main axis) coincides with the crystallographic b axis. Therefore, other two optic main axes are in the ac plane.

Thus, it was identified that MNBA-Et crystal useful as a second-order nonlinear optical medium has the above-described structure and optical properties.

Example 2

Measurement of Second-order Nonlinear Susceptibility ($X^{(2)}$) by Maker Fringe Method

The second-order nonlinear optical effects and their anisotropy of the MNBA and MNBA-Et crystals having the specific structure thus prepared were examined by the Maker fringe method (Electron. Lett., 23-(11), 595(1987)), which is a method for measuring the directional dependence of the SHG intensities, using plate-shaped single crystals, using Nd:YAG laser (1064 nm).

A plate-shaped single crystal of MNBA or MNBA-Et with a thickness of about 100 μm, in which the (0 1 0) face is the largest face, was set in a goniometer. The relationship between the rotation angle $\theta$ about the axis of a linearly polarized laser beam (Nd:YAG laser, 1064 nm) impinging normal to the (0 1 0) face and the observed SHG intensities was determined. Also, the relationship between the incident angle $\phi$ of the laser beam to the (0 1 0) face and the SHG intensities was determined.

The $\theta$ dependence was nearly a $\cos^2\theta$ function with a period of $\pi$, and the $\phi$ dependence was so called the fringe pattern.

In either of the MNBA and MNBA-Et plate-shaped single crystals, the largest second-order nonlinear susceptibility was

$$X^{(2)}_{11}$$

obtained when the linear polarization (polarized plane) of the impinging light was coincident with the x axis in the (0 1 0) face.

The second-order nonlinear susceptibilities

$$\chi^{(2)}_{11}$$

X of the MNBA and MNBA-Et crystals were about 5.0 x $10^{-6}$ esu and about 3.9 x $10^{-6}$ esu, respectively, which are about 4 times and about 3 times, respectively, as large as that of the MNA crystal which has the largest second-order nonlinear susceptibility (1.2 x $10^{-6}$ esu) among the conventional nonlinear optical media.

Example 3

Preparation of Thin Film Single Crystals of MNBA and MNBA-Et Having Optically Smooth (0 1 0) Face

A solution of MNBA (about 12 wt%) in dimethylformamide (DMF) was prepared at 60° C and the solution was sandwiched between a pair of glass plates which had been polished to have optical smoothness. The resultant was left to stand in a thermostated atmosphere at 53° C. After about 10 days, thin film single crystals were grown. The largest one sized 25 mm x 4 mm x 5 μm.

A solution of MNBA-Et (about 9 wt%) in DMF was prepared and the solution was processed in the same manner as in the processing of the MNBA solution just mentioned above. Thin single crystals of MNBA-Et, whose largest size was 20 mm x 4 mm x 5 μm, were grown.

X-ray diffraction analysis revealed that in either of the MNBA and MNBA-Et crystals, the grown smooth surface parallel to the glass plate was (0 1 0) face. The morphology of the MNBA-Et thin film single crystal is shown in Fig. 2.

By observing the interference images with a conoscope, it was confirmed that in either of the MNBA and MNBA-Et thin film single crystals, one of the optic main axes is perpendicular to the surface parallel to the glass plates.

One of the glass plates was peeled off so as to expose a smooth (0 1 0) face. The thin film single crystals having a glass plate at one side may be used as they are as second-order waveguiding type nonlinear optical elements. A linearly polarized light of He-Ne laser was guided in the TE mode. In this case, the substantially optically smooth surfaces with respect to waveguiding were (0 1 0) face and (0 -1 0) face. From the good waveguiding conditions, it was confirmed that these thin film single crystals can utilize the largest second-order nonlinear optical coefficient.

Example 4

Preparation of Optical Modulator Utilizing Electro-optic Element and Measurement of linear Electro-optic Effect

By utilizing the (0 1 0) face (or the backside, (0 -1 0) face which is equivalent to (0 1 0) face) of the thin film single crystals of MNBA or MNBA-Et prepared in Example 3 having the substantially optically smooth surface as electro-optic elements, optical modulators were constructed, and the linear electro-optic effects and their anisotropic dependency on the electric field was examined.

Referring to Fig. 3a, a thin film single crystal 31 was adhered to a glass plate 32 in such manner that the smooth (0 -1 0) face contacts the glass plate 32. Aluminum parallel electrodes 34 (thickness: about 200 nm; distance between the electrodes: 5 μm; width of electrode: 1 mm; length of electrode: 40 mm) formed on a glass plate 33 by vapor-deposition of aluminum and subsequent patterning, were pressed to the (0 1 0) face to construct an electrooptic device 3. A linearly polarized light of He-Ne laser beam (633 nm) obtained by passing through a polarizer was impinged on the device in such manner that the linear polarization is on the (0 1 0) face (on the ac plane). The light was passed through a Soleil-Babinet's compensator 35 to compensate the intrinsic birefringence, and the intensity of the output light from an analyzer 39 perpendicular to the polarizer 38 was measured by a photodetector 36. To the parallel electrodes 34, a sinusoidal voltage of 10 kHz was applied by a function generator 37. The relationship between the signal and the sinusoidal voltage, which was drawn on an oscilloscope is shown in Fig. 3b.

The anisotropic dependence of the linear electro-optic effect on the applied electric field was measured by intermittently rotating the electric field from the direction coincident with the x axis with an interval of 15° by rotating the glass plate 33 which has the parallel electrodes on the (0 1 0) face. As a result, the

largest linear electro-optic effect was obtained when the direction of the electric field was coincident with the x axis.

Using the relationships expressed by the formulae (1), (2) and (3) mentioned earlier, the figure of merit $|n_1{}^3r_{11} - n_3{}^3r_{31}|/2$ was calculated. The figure of merit for MNBA and MNBA-Et crystals were as large as about 930 pm/V and about 700 pm/V, which are about 8 times and about 6 times larger than that of the LN crystal (120 pm/V), respectively, or which are about 3 times and about 2.3 times, respectively, larger than that of the MNA crystal ($n_1{}^3r_{11}/2$).

Example 5

Preparation of SHG Device

An example of an SHG device utilizing a plate-shaped single crystal of MNBA or MNBA-Et crystal will now be described.

Referring to Fig. 4, on the (0 1 0) face of a plate-shaped single crystal, tantalum pentoxide ($Ta_2O_5$) was vapor-deposited to a thickness of about 800 - 900 nm to form a passive planar waveguide 42. The constitution of a device 4 utilizing the plate-shaped single crystal of MNBA-Et is shown in Fig. 4. In this constitution, the refractive index of tantalum pentoxide is higher than that of the plate-shaped single crystal at the wavelength of the Nd:YAG laser and lower at the wavelength of the second harmonic generation.

Into the passive planar waveguide 42, a linearly polarized light of Nd:YAG laser was impinged in such manner that its plane of polarization was in parallel to the (0 1 0) face. In other words, the linearly polarized light was impinged and propagated in the planar waveguide in the TE mode. The power density of the impinging Nd:YAG laser was about 1 $MW/cm^2$.

Generation of a green SH light and radiation to the crystal side was observed. The maximum SH light was observed when the plane of polarization of the Nd:YAG laser was coincident with the x axis.

Example 6

An example of an electro-optic device (branched interferometer type optical modulator) comprising MNBA crystal, which has a three dimensional waveguide (channel waveguide) will now be described referring to Fig. 5.

On the (0 1 0) face of a thin film single crystal (20 mm x 4 mm x 0.8 $\mu$m) of MNBA having a glass plate on one side, which was prepared in the same manner as in Example 3, a posi-type photoresist (Shipley MP1400) was applied using a spinner and was exposed to light through a chromium mask having a pattern of the waveguide. The photoresist was then developed and the patterning of the crystal was carried out by reactive ion etching (RIE) at low temperature. Finally, the entire surface was exposed to light and the photoresist was made to be soluble and removed. In this process, the chromium pattern was set so that the direction of the waveguide is perpendicular to the axis 51 of absorbing in the longest wavelengths, that is, so that the polarized plane of the light is coincident with the axis of absorbing in the longest wavelengths.

Then silicon dioxide ($SiO_2$) 58 was vapor-deposited on the entire surface to a thickness of about 600 nm by electron beam vapor-deposition under the oxygen atmosphere.

Then aluminum was vapor-deposited on the entire surface to a thickness of about 200 nm.

A posi-type photoresist was applied on the entire surface and was exposed to light through a chromium mask having a pattern of the electrodes, followed by development of the photoresist. The aluminum layer was etched by an aqueous solution of potassium hydroxide (2 wt%)/potassium ferricyanide (1 wt%) to form planar electrodes 57.

A surface for impinging or emitting of a light, which is constituted by an end face of the glass plate and cleavage face of the waveguide of the MNBA crystal, wherein cleavage face is flush with the end face of the glass plate, was provided to the layered structure of glass plate/MNBA waveguide/$SiO_2$. This was carried out by notching the glass plate from the backside in the direction of the cleavage of the MNBA (this direction is identical with the direction of the axis of absorbing in the longest wavelengths) so that the last minute portion of the glass plate in the direction of its thickness remains intact. Then the glass plate is pulled off so as to separate it at the notch together with the crystal and $SiO_2$ layer formed thereon.

The branched interferometer type optical modulator 5 thus prepared is shown in Fig. 5a and Fig. 5b (cross sectional view).

The light path length (the length of the light controlling waveguide 54) was 1 mm, which was 1/6 of that of the conventional optical modulator using the LN crystal.

A linearly polarized light of He-Ne laser (633 nm) was guided in the TM mode. When no voltage is

applied, strong scattering light was observed at the end face of the waveguide. Increasing the applied dc voltage, the scattering light at the end face of the waveguide became not observable, at this time the voltage was about 2.8V. Thus, in this device, the $V_\pi$ was found to be about 2.8V for the dc voltage.

An ac voltage was applied in 10 MHz which was the high frequency limit of the pulse generator employed and the intensity of the emitted light was monitored by a photodetector. When the ac voltage was about 3V, the wave shown in Fig. 6 was observed on an oscilloscope. As a result, it was confirmed that the device prepared can function without problem in the range of this frequency.

By comparing the results obtained with those of the branched interferometer type optical modulators utilizing the LN crystal, it is apparent that the optical modulator according to the present invention may be made smaller and/or the driving voltage may be lower than the conventional optical modulators using the LN crystal.

Example 7

In the similar manner to Example 6, a total internal reflection switch 7 shown In Fig. 7 was prepared.

That is, two MNBA waveguide 71 with a width of about 10 μm were crossed at a crossing angle of about 7.5°, and planar electrodes 72 were formed at the crossing portion to form a switching portion. The distance between the electrodes 74 was about 4 μm. Thus, the axis 73 of absorbing in the longest wavelengths of the MNBA crystal was perpendicular to the longitudinal direction of the planar electrodes.

A linearly polarized light of He-Ne laser beam obtained by passing through a polarizer was impinged horizontally on the end face 7a of the waveguide and was guided in the TE mode.

When no voltage was applied, it was confirmed by observing the red light scattered at the end face that the light went straight and was emitted from an end face 7a'.

Then a dc voltage of 5V was applied to the planar electrodes. The red scattered light was observed on an end face 7b' of the waveguide, which was opposing one to the end face 7a' where the red scattered light was observed when no voltage was applied. Thus, it was confirmed that with this device, the light path can be switched by applying a voltage to the electrodes.

The similar TIR switch employing the LN crystal has been reported (C.S. Tsai et al., "Optical channel waveguides switch and coupler using total internal reflection", IEEE J. Quantum Electron., vol. QE-14, No. 7, pp. 513-517, July 1978). Comparing the present device with the reported switch, the crossing angle of the switch utilizing MNBA crystal is about twice as large as that of the reported switch employing the LN crystal. Therefore, it has been shown that the degree of integration of an optical switch of the branched type made of MNBA crystal can be about four times higher than that of LN crystal.

Although the present invention was described by way of preferred embodiments thereof, it is apparent for those skilled in the art that various modifications may be made without departing from the spirit and scope of the present invention.

**Claims**

**1.** A second-order nonlinear optical device comprising an optical element of non-centerosymmetric monoclinic crystal of 4'-nitrobenzylidene-3-alkanoylamino-4-methoxyaniline of the formula

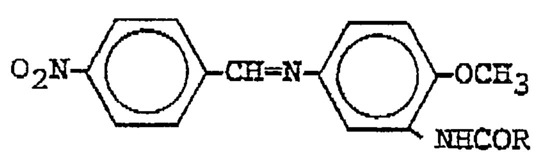

(wherein R represents a $C_1$ or $C_2$ alkyl or haloalkyl) or a derivative thereof in which at least one hydrogen atom is substituted with deuterium atom, said monoclinic crystal belonging to space group Cc, point group #9, said optical element having at least one substantially optically smooth surface for impinging, transmitting, and/or propagating a light having a main component of the optical wave with polarization along the axis of absorbing in the longest wavelengths, said axis being in the ac plane of said monoclinic crystal.

**2.** The second-order nonlinear optical device of claim 1, wherein said substantially optically smooth surface is (0 1 0) face of the monoclinic crystal.

3. The second-order nonlinear optical device of claim 1, which is of waveguide type.

4. The second-order nonlinear optical device of claim 3, further comprising at least a pair of electrodes for applying an electric field having a large component substantially in the direction of said axis of absorbing in the longest wavelengths in the ac plane of said monoclinic crystal.

5. The second-order nonlinear optical device of claim 4, wherein said substantially optically smooth surface is (0 1 0) face of the monoclinic crystal, and said pair of electrodes are provided on (0 1 0) face.

6. The second-order nonlinear optical device of claim 1, wherein said monoclinic crystal is a crystal of 4'-nitrobenzylidene-3-acetoamino-4-methoxyaniline.

7. The second-order nonlinear optical device of claim 3, wherein said monoclinic crystal is a crystal of 4'-nitrobenzylidene-3-acetoamino-4-methoxyaniline.

8. The second-order nonlinear optical device of claim 4, wherein said monoclinic crystal is a crystal of 4'-nitrobenzylidene-3-acetoamino-4-methoxyaniline.

9. The second-order nonlinear optical device of claim 1, wherein said monoclinic crystal is a crystal of 4'-nitrobenzylidene-3-ethylcarbonylamino-4-methoxyaniline.

10. The second-order nonlinear optical device of claim 3, wherein said monoclinic crystal is a crystal of 4'-nitrobenzylidene-3-ethylcarbonylamino-4-methoxyaniline.

11. The second-order nonlinear optical device of claim 4, wherein said monoclinic crystal is a crystal of 4'-nitrobenzylidene-3-ethylcarbonylamino-4-methoxyaniline.

FIG. 1a

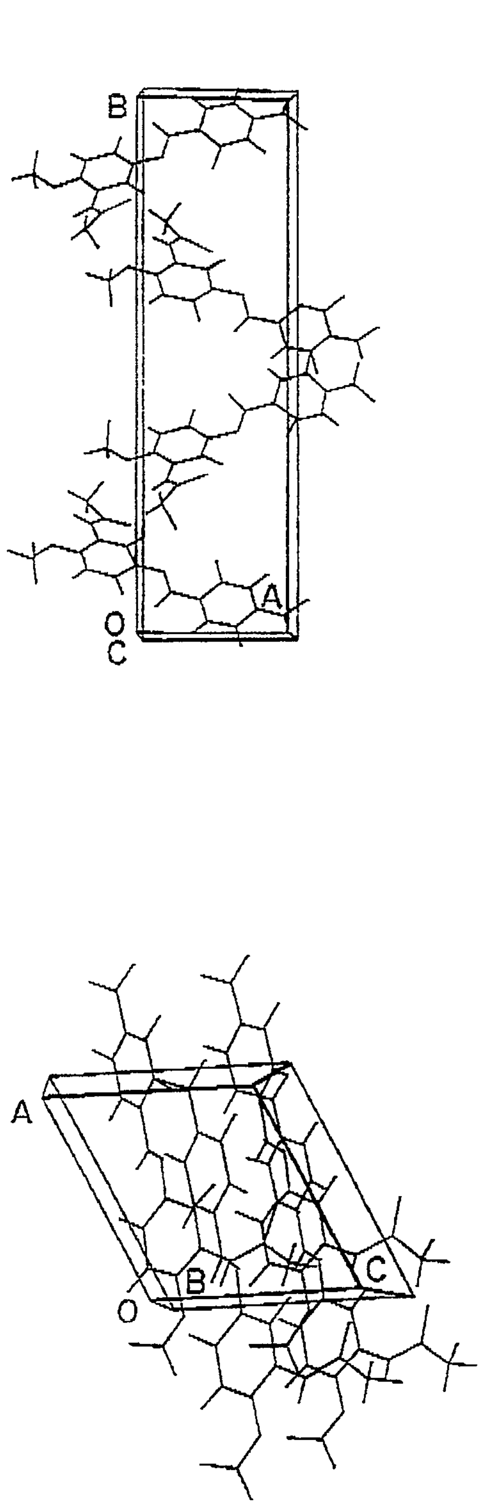

FIG. 1b

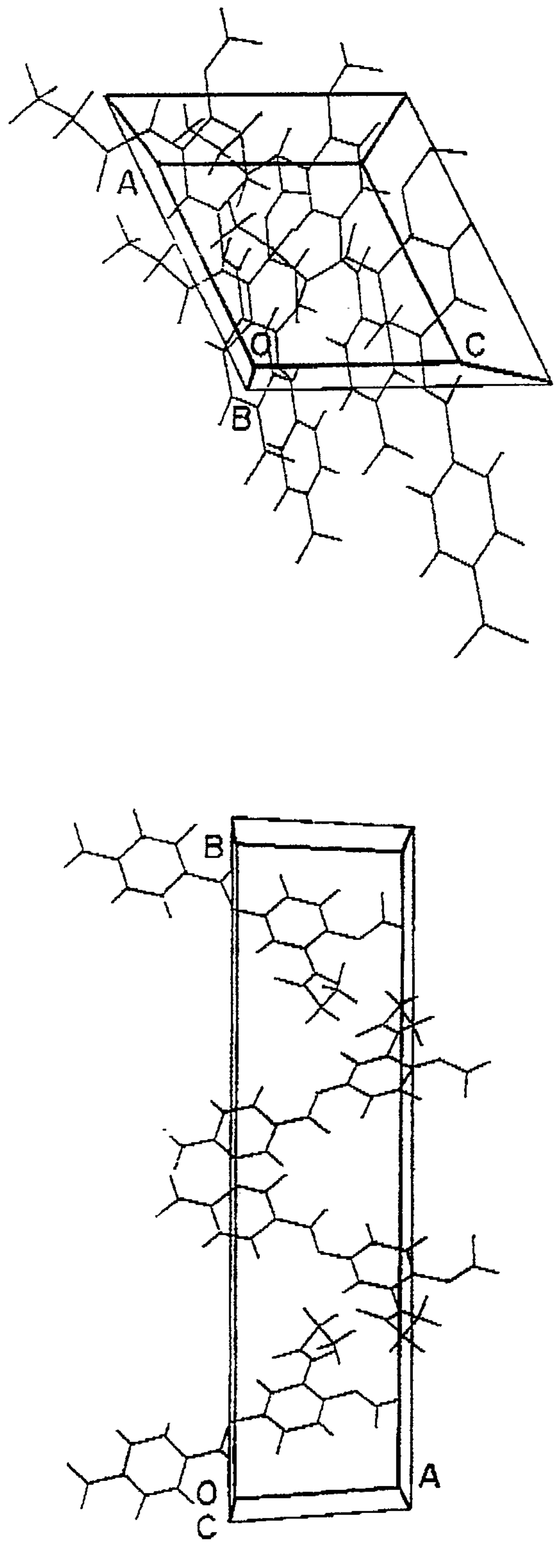

FIG. 2

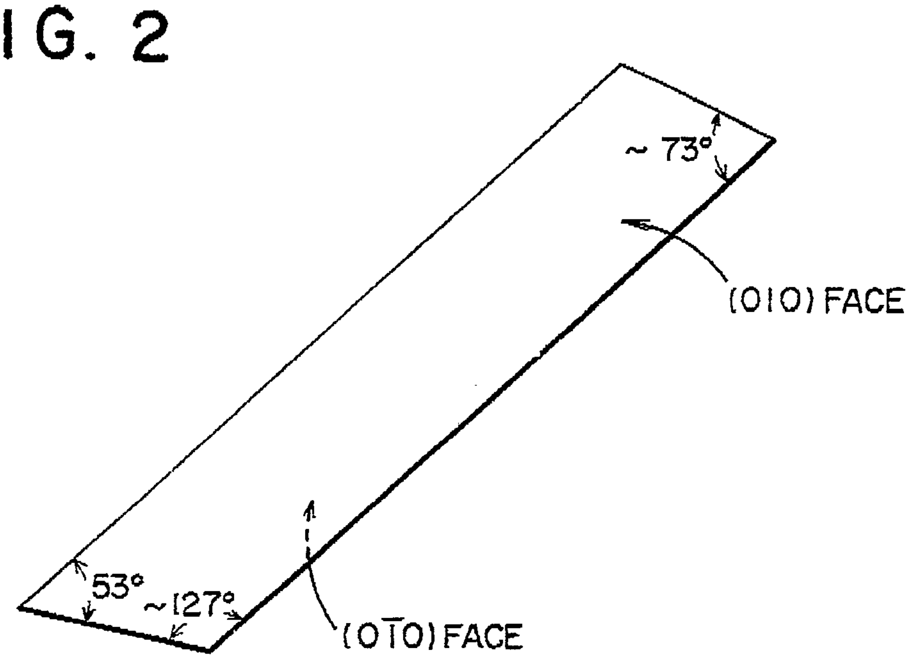
~ 73°
(010) FACE
53°
~127°
(0$\bar{1}$0) FACE

FIG. 3a

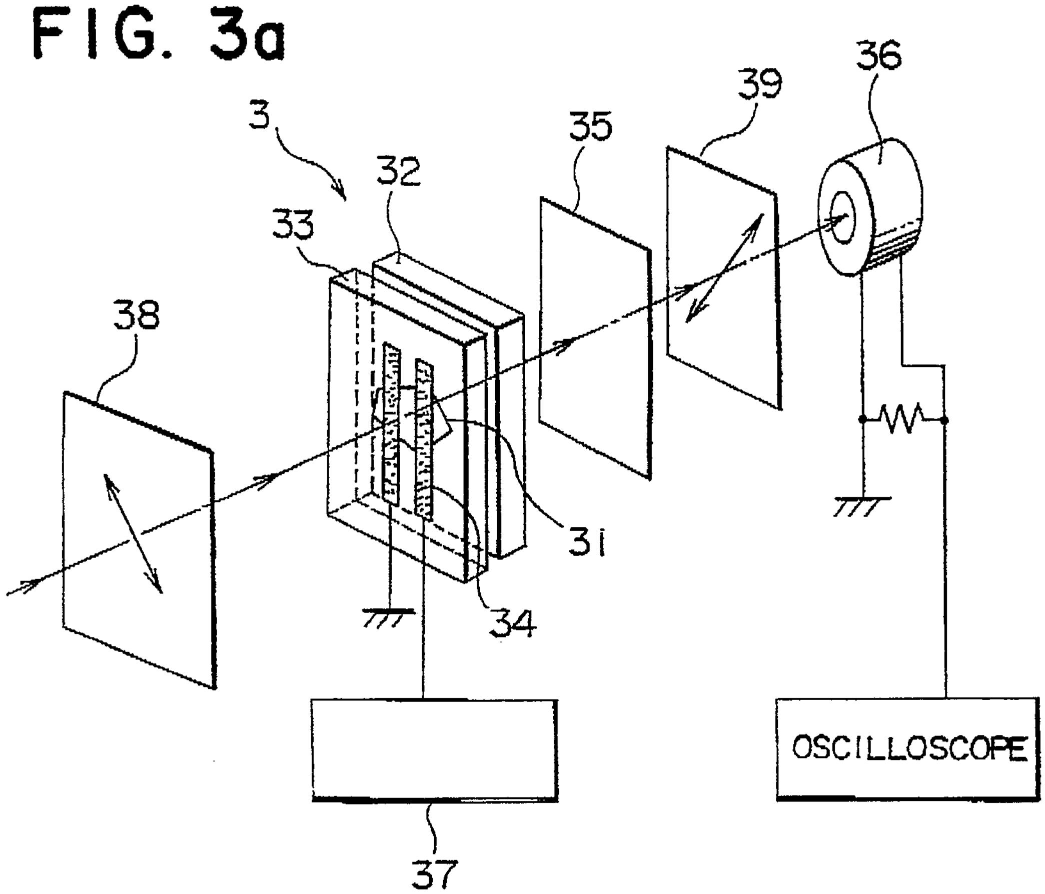
3
32
33
35
39
36
38
31
34
37
OSCILLOSCOPE

F I G. 3b

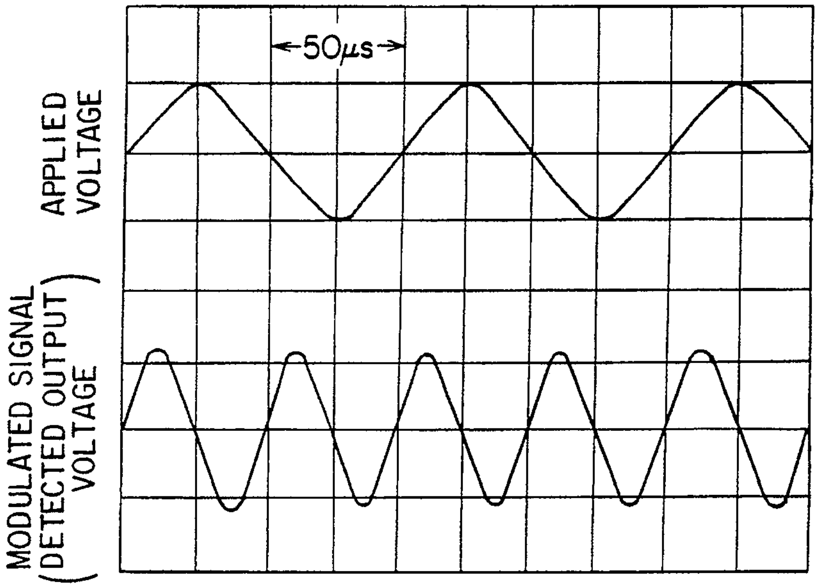
50μs
APPLIED VOLTAGE
MODULATED SIGNAL (DETECTED OUTPUT VOLTAGE)

F I G. 4

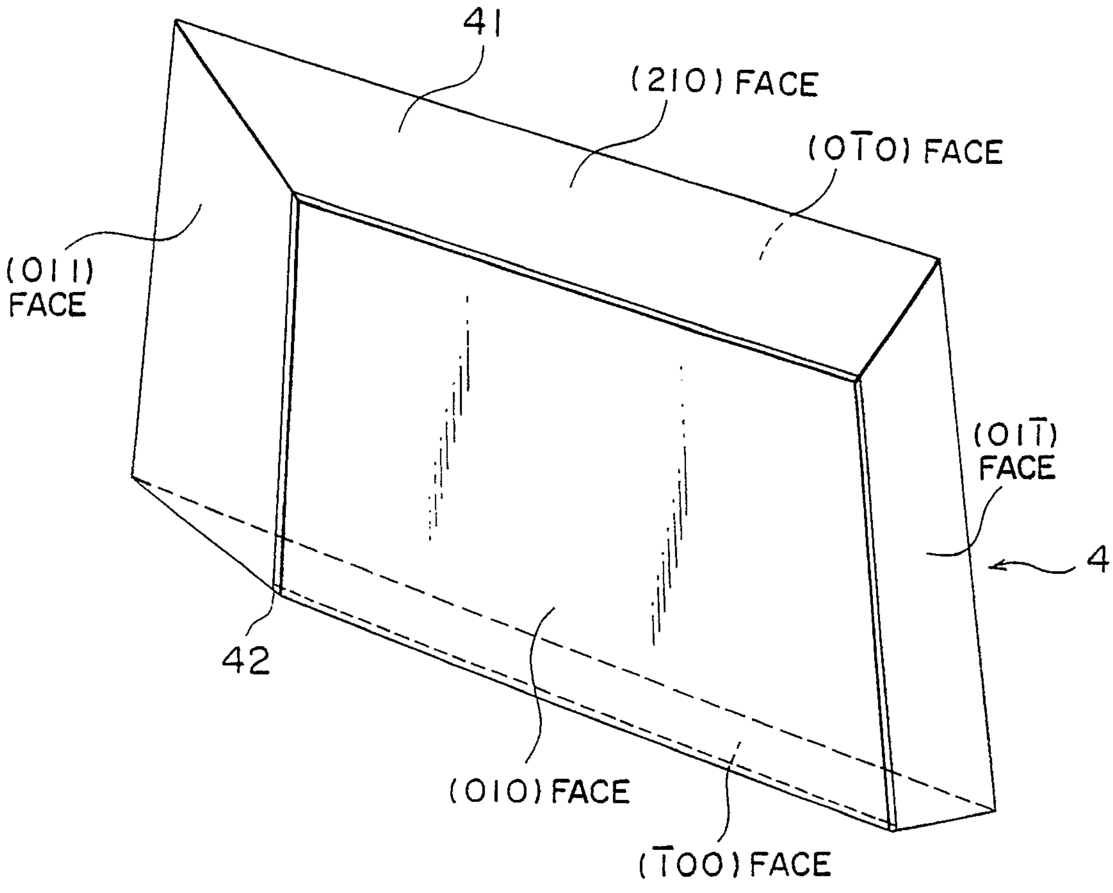
41
(210) FACE
(0$\bar{1}$0) FACE
(011) FACE
(01$\bar{1}$) FACE
4
42
(010) FACE
($\bar{1}$00) FACE

# FIG. 5a

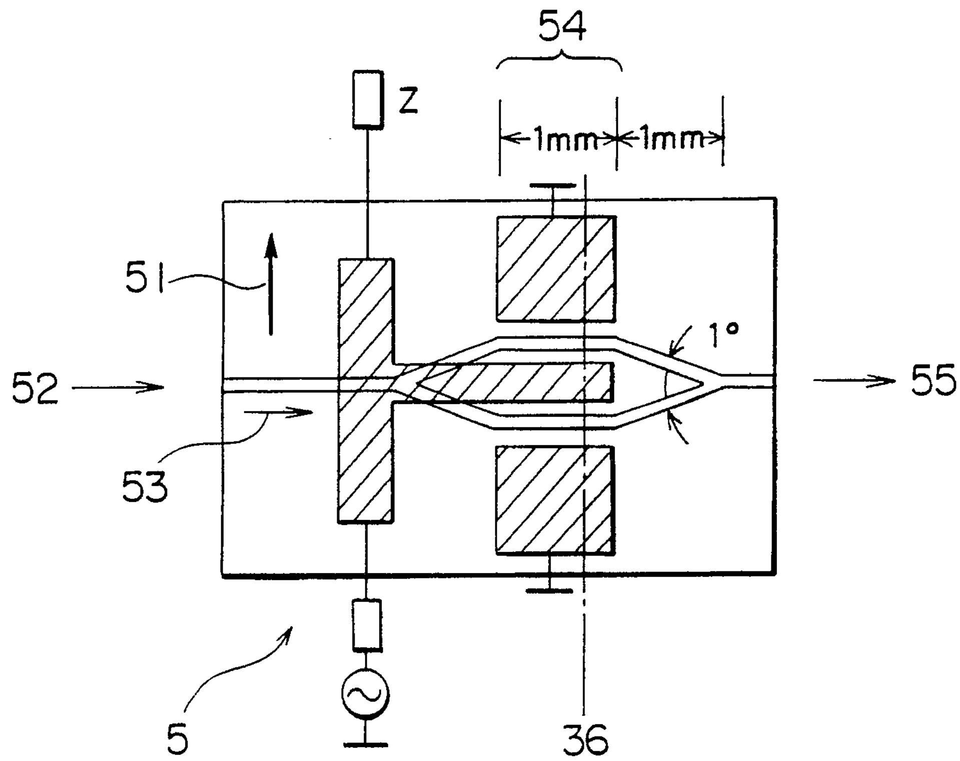


# FIG. 5b

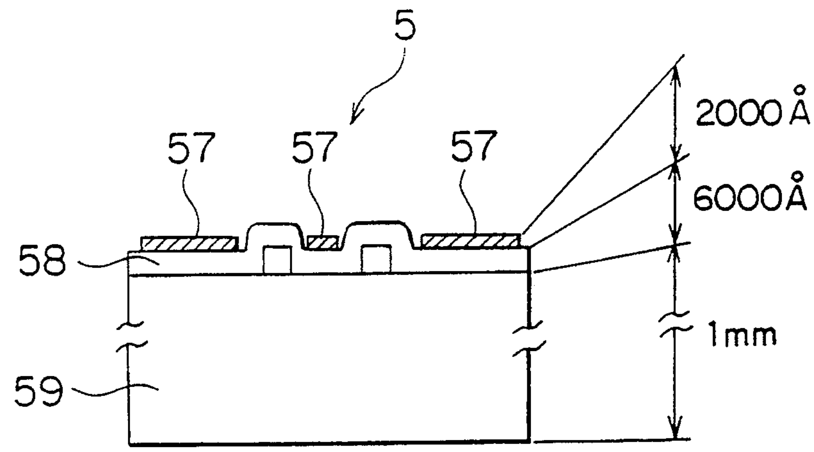

FIG. 6

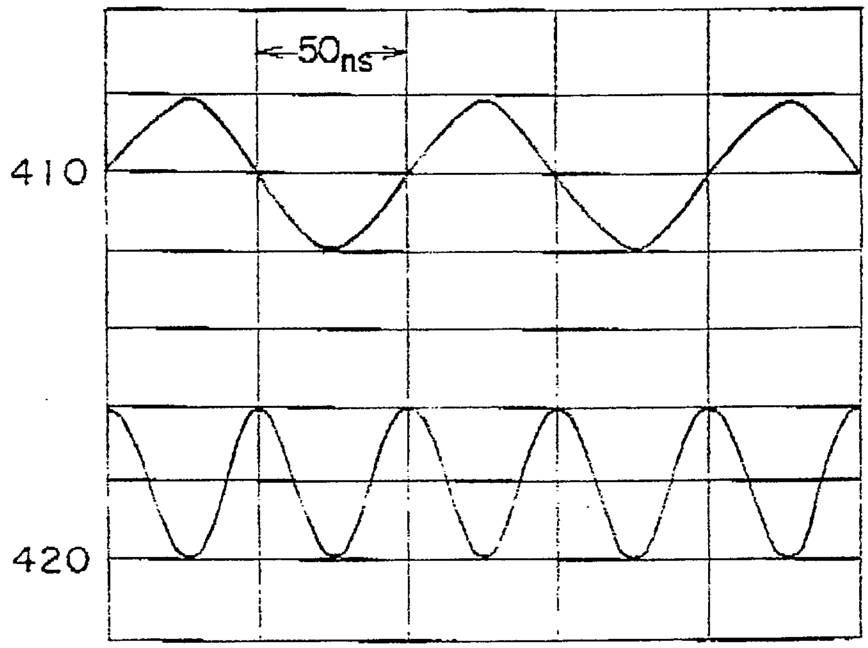


FIG. 7

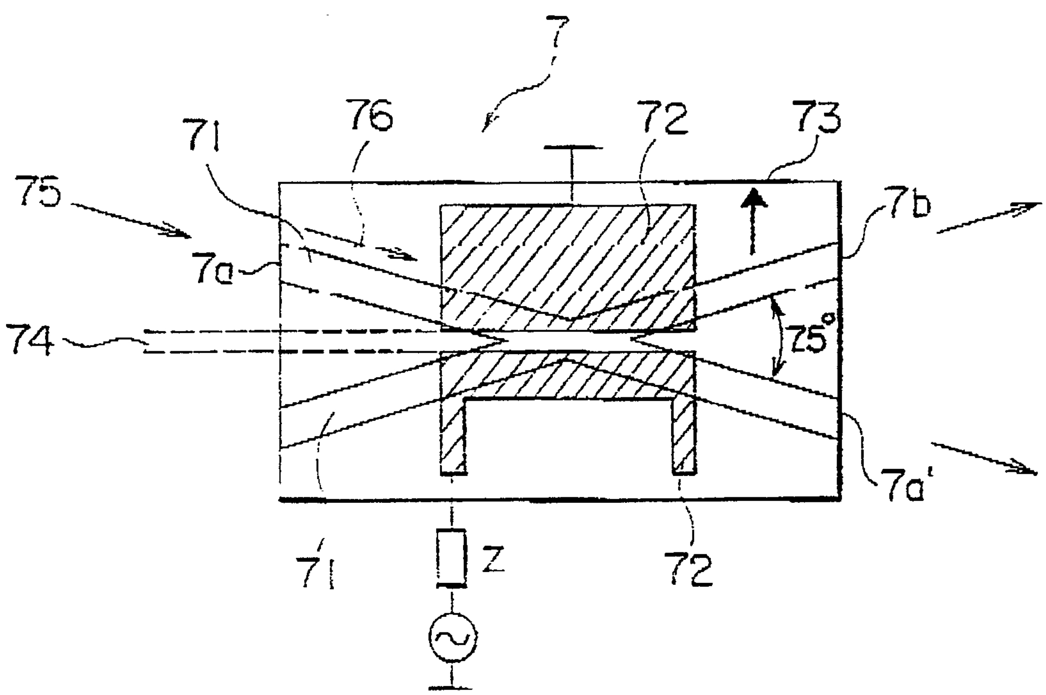

FIG. 8a

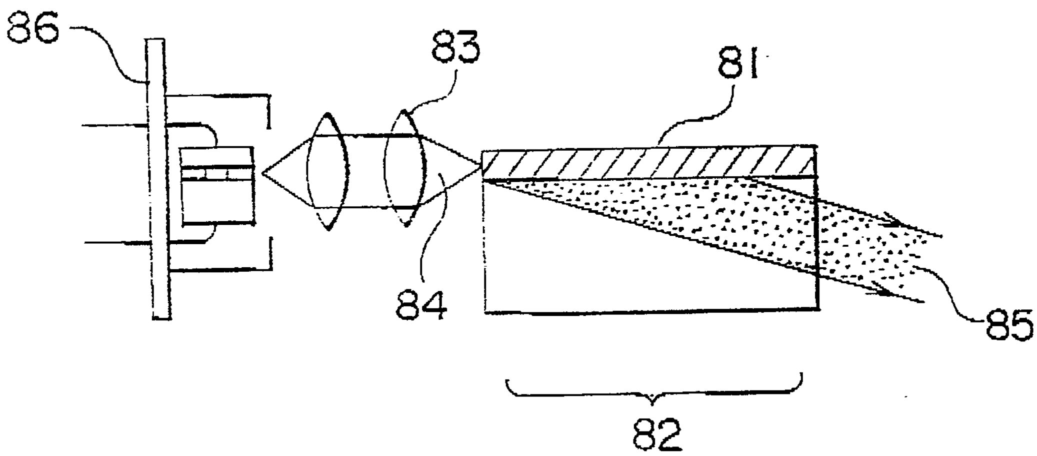


FIG. 8b

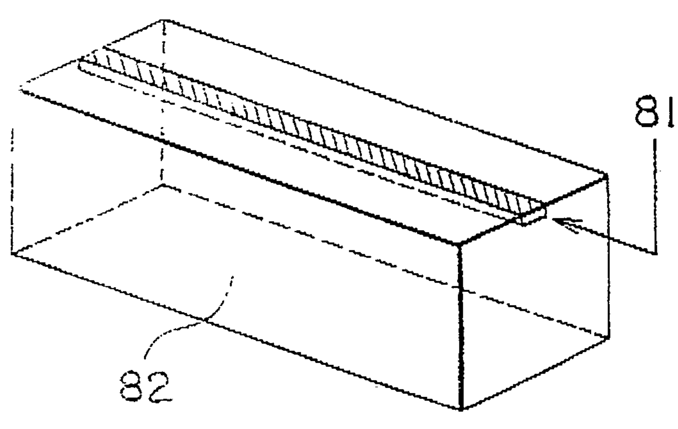

FIG. 9

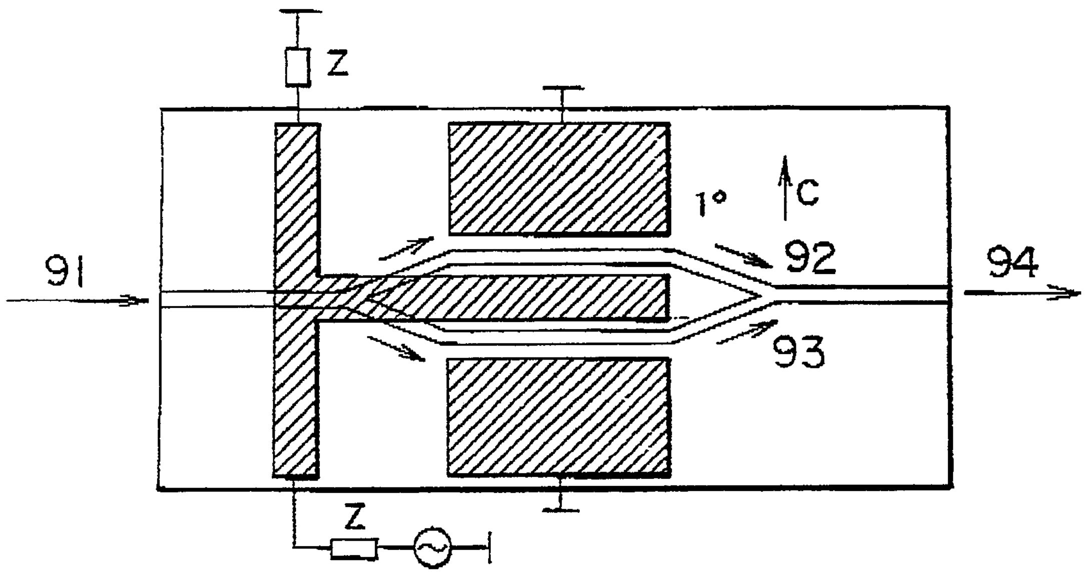


FIG. 10

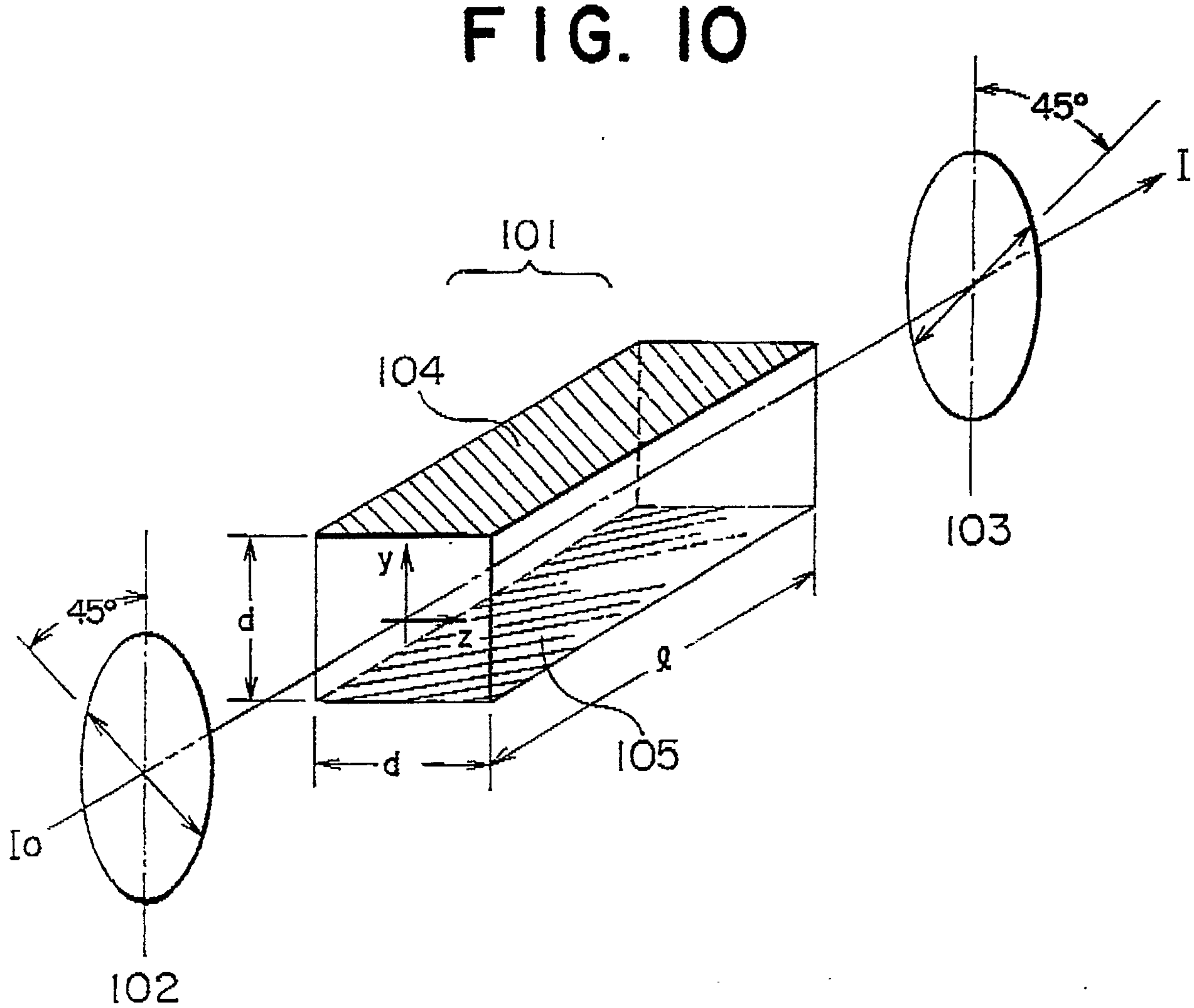

# INTERNATIONAL SEARCH REPORT

International Application No PCT/JP90/00846

**I. CLASSIFICATION OF SUBJECT MATTER** (if several classification symbols apply, indicate all) [6]

According to International Patent Classification (IPC) or to both National Classification and IPC

Int. Cl$^5$ G02F1/35, C07C251/24

**II. FIELDS SEARCHED**

Minimum Documentation Searched [7]

| Classification System | Classification Symbols |
|---|---|
| IPC | G02F1/35 |

Documentation Searched other than Minimum Documentation to the Extent that such Documents are Included in the Fields Searched [8]

| | |
|---|---|
| Jitsuyo Shinan Koho | 1966 - 1990 |
| Kokai Jitsuyo Shinan Koho | 1971 - 1990 |

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| X,P | JP, A, 2-66524 (Konica Corp), 6 March 1990 (06. 03. 90), Upper left column, page (3) | 1 - 11 |
| X,P | JP, A, 1-200235 (Toray Industries, Inc.), 11 August 1989 (11. 08. 89), Pages (2) and (4) | 1 - 11 |
| A | JP, A, 64-38734 (Sumitomo Electric Industry Ltd.), 9 February 1989 (09. 02. 89) | 1 - 11 |
| X | JP, A, 63-113429 (Toray Industries, Inc.), 18 May 1988 (18. 05. 88) & EP, A, 250099 | 1 - 11 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| September 5, 1990 (05. 09. 90) | September 17, 1990 (17. 09. 90) |
| **International Searching Authority** | **Signature of Authorized Officer** |
| Japanese Patent Office | |